# EUROPEAN PATENT SPECIFICATION

(11) **EP 3 316 851 B1**
(45) Date of publication and mention of the grant of the patent: **11.09.2019**
(21) Application number: 16726901.8
(22) Date of filing: 06.06.2016
(51) Int. Cl.: A61K 8/24, A61K 8/26, A61Q 11/00, A61K 8/19

(54) **ORAL CARE COMPOSITION COMPRISING CALCIUM SILICATE**
MUNDPFLEGEZUSAMMENSETZUNG ENTHALTEND KALCIUMSILIKAT
COMPOSITION DE PROTECTION ORALE COMPRENANT DU SILICATE DE CALCIUM

(30) Priority: 03.07.2015 WO PCT/CN2015/083268; 05.08.2015 EP 15179783
(43) Date of publication of application: 09.05.2018
(73) Proprietor: Unilever NV, 3013 AL Rotterdam (NL); Unilever PLC, London, Greater London EC4Y 0DY (GB)
(72) Inventor: JOINER, Andrew, Wirral Merseyside CH63 3JW (GB); LI, Xiaoke, Shanghai 200335 (CN); LIU, Weining, Shanghai 200335 (CN)
(74) Representative: Tansley, Sally Elizabeth
(86) International application number: PCT/EP2016/062777
(87) International publication number: WO 2017/005431

(56) References cited:
- WO-A2-2014/056713
- CN-A- 103 893 038
- US-A1- 2013 251 772
- DATABASE GNPD [Online] MINTEL; September 2014 (2014-09), "dental calming kit for dogs", XP002751301, Database accession no. 2589555

## Description

### Technical Field of the Invention

The present invention relates to oral care compositions such as tooth pastes, gums, mouthwashes and the like. In particular the present invention relates to an oral care composition containing calcium silicate and a deposition aid that results in remineralization and/or reducing sensitivity and/or whitening of teeth. The invention also relates to the use of such composition for remineralizing and/or reducing sensitivity and/or whitening of teeth of an individual.

### Background of the Invention

The enamel layer of the tooth is naturally an opaque white or slightly off-white colour. However, this enamel layer can become stained or discoloured.

Many products we consume have a negative impact on our teeth and mouth. Acidic drinks and sweets, for example, can result in tooth erosion by attacking enamel which is the outer coating that protects the teeth. Moreover, tobacco based products as well as beverages like coffee and tea can stain or reduce the whiteness of one's teeth. These staining and discolouring substances are often able to permeate the enamel layer. This problem occurs gradually over many years, but imparts a noticeable discoloration of the enamel of one's teeth.

In addition to what we consume, the natural equilibrium between tooth hydroxyapatite being dissolved from the enamel of teeth and hydroxyapatite being formed on or in teeth from substances occurring naturally in the saliva shifts continuously. Such a shift can yield unattractive teeth with cariogenic conditions. A variety of products are currently used for addressing tooth decay and/or tooth whitening. Such products often comprise peroxides, abrasives or both. These types of products are often not desired since they do not contribute to the remineralization of teeth and can cause damage to teeth and gums if overused. Products comprising calcium source have been developed in an attempt to enhance tooth remineralization.

Tooth hypersensitivity is a temporary induced pain sensation that affects up to 20% of the adult population. Hypersensitive teeth may be sensitive to temperature, pressure or chemical action.

The dentin of the tooth generally contains channels, called tubules, which provide for an osmotic flow between the inner pulp region of the tooth and the outer root surfaces. Tooth hypersensitivity may be related to the general increase in exposed root surfaces of teeth as a result of periodontal disease, toothbrush abrasion, or cyclic loading fatigue of the thin enamel near the dento-enamel junction. When root surfaces are exposed, dentinal tubules are also exposed.

The currently accepted theory for tooth hypersensitivity is the hydrodynamic theory, based on the belief that open exposed dentinal tubules allow fluid flow through the tubules. This flow excites the nerve endings in the dental pulp. Clinical replica of sensitive teeth viewed in a SEM (scanning electron microscopy) reveal varying numbers of open or partially occluded dentinal tubules.

There are different approaches to treat tooth hypersensitivity. One approach is to reduce the excitability of the nerve in a sensitive tooth by using "nerve-depolarising agents" comprising strontium ions, potassium salts such as potassium nitrate, potassium bicarbonate, potassium chloride and the like. These nerve-depolarising agents function by interfering with neural transduction of the pain stimulus to make the nerve less sensitive.

Another approach is to use "tubule blocking agents" that fully or partially occlude tubules such as polystyrene beads, apatite, polyacrylic acid, mineral hectorite clay and the like. These tubule blocking agents function by physically blocking the exposed ends of the dentinal tubules, thereby reducing dentinal fluid movement and reducing the irritation associated with the shear stress described by the hydrodynamic theory.

The present inventors have now recognized a need to develop an oral care product which is suitable to remineralize and/or reduce sensitivity and/or whiten teeth which is also gentle in use. It has been found unexpectedly that the tooth remineralization efficacy can be enhanced by using calcium silicate in combination with certain deposition aids. Besides, the present inventors have further found that such an oral care composition is effective in blocking dentinal tubules to reduce tooth sensitivity which is beneficial for treating tooth hypersensitivity. Additionally, such a composition unexpectedly enhances the deposition of particulate tooth whitening agent on tooth surfaces to provide tooth whitening benefit.

### Additional Information

WO 2008/068149 A (Unilever) discloses an oral care product comprising a first composition comprising an insoluble calcium salt that is not a calcium phosphate salt, a second independent composition comprising a source of phosphate ions, and a means for delivering each of the compositions to the surface of the teeth. The preferred insoluble calcium salt is calcium silicate.

WO 2014/056713 (Unilever) discloses oral care compositions comprising calcium silicate hydrate comprising from 20 to 70% SiO₂ by weight of the calcium silicate hydrate, and physiologically acceptable carrier comprising humectants, surfactant, thickener or a mixture thereof.

DATABASE GNPD [Online] MINTEL; September 2014 (2014-09), "dental calming kit for dogs", Database accession no. 2589555 discloses a dental calming kit for dog to be brushed on the teeth, i.e. an oral care composition. It comprises a calcium silicate (calcium sodium silicate) and calcium sulfate and dicalcium phosphate.

CN 103 893 038 A (Haian Yatai Additives Co Ltd) discloses a toothpaste which is prepared by mixing the following components by weight: 10-15 parts of calcium bicarbonate, 10-15 parts of calcium silicate, 1-1.5 parts of calcium hydrogen phosphate, 20-30 parts of sodium hydroxide monohydrate, 6-10 parts of alumina, 4-6 parts of citric acid, 4-6 parts of clove oil, 0.5-1.5 parts of sodium lauryl sulfonate, 0.5-1.5 parts of sodium phosphate, 1-2 parts of sorbitol, 0.1-0.5 parts of xylitol, 4-6 parts of dioxo phenyl guanidine radical and 30-40 parts of deionized water.

US 2013/251772 A1 (Colgate) discloses an oral care composition includes an effective amount of calcium silicate particles. The calcium silicate particles have an average diameter of less than about 5 microns.

None of the references above describes an oral care composition comprising calcium silicate and a deposition aid in a weight ratio from 20:1 to 1:5, particularly when the deposition aid is selected from calcium sulfate hemihydrates, calcium dihydrogen phosphate or mixtures thereof.

### Tests and Definitions

### Dual-Phase

"Dual-Phase" for the purposes of the present invention means a composition having two independent phases which are physically separate.

### Dentifrice

"Dentifrice" for the purposes of the present invention means a paste, powder, liquid, gum or other preparation for cleaning the teeth or other surfaces in the oral cavity.

### Tooth Paste

"Tooth paste" for the purpose of the present invention means a paste or gel dentifrice for use with a toothbrush. Especially preferred are tooth pastes suitable for cleaning teeth by brushing for about two minutes.

### Mouth Wash

"Mouth wash" for the purpose of the present invention means liquid dentifrice for use in rinsing the mouth. Especially preferred are mouth washes suitable for rinsing the mouth by swishing and/or gargling for about half a minute before expectorating.

### Particle Size

"Particle size", as used herein, refers to particle diameter unless otherwise stated. Diameter is meant to mean the largest measurable distance or a leading dimension on a particle in the event a well-defined sphere is not generated. Particle size can be measured, for example, by dynamic light scattering (DLS).

### Composite Particle

"Composite particle", as used herein, refers to a particle comprising a first component core and a second component coating wherein the core and coating are composed of different materials.

### Refractive Index

Refractive index is quoted at a temperature of 25°C and a wavelength of 589 nm.

### Solubility

"Soluble" and "insoluble", as used herein, refers to the solubility of a source (e.g., like calcium salts) in water at 25°C and atmospheric pressure. "Soluble" means a source that dissolves in water to give a solution with a concentration of at least 0.1 moles per litre. "Insoluble" means a source that dissolves in water to give a solution with a concentration of less than 0.001 moles per litre. "Slightly soluble", therefore, is defined to mean a source that dissolves in water to give a solution with a concentration of higher than 0.001 moles per litre and less than 0.1 moles per litre.

### Water of Hydration

"Water of hydration", as used herein, refers to water chemically combined with a substance in a way that it can be removed by heating without substantially changing the chemical composition of the substance. In particular, water which could only be removed when heated above 200°C. The water loss is measured using thermo gravimetric analysis (TGA) with a Netzsch TG instrument. The TGA is conducted under an N₂ atmosphere with heating rate of 10 degree/min in the range of 30 to 900°C.

### Substantially Free

"Substantially free of', as used herein, means less than 1.5%, and preferably less than 1.0%, and more preferably less than 0.75% and more preferably still less than 0.5%, and even more preferably less than 0.1% and most preferably from 0.0 to 0.01% by weight, based on total weight of the oral care composition, including all ranges subsumed therein.

### Hydrous Composition

"Hydrous composition", as used herein, means the composition comprises water higher than 1.5%, preferably higher than 5%, more preferably higher than 10% and most preferably from 20 to 90% by weight of the composition.

### Viscosity

Viscosity of a tooth paste is the value taken at room temperature (25°C) with a Brookfield Viscometer, Spindle No.4 and at a speed of 5 rpm. Values are quoted in centipoises (cP=mPa.s) unless otherwise specified.

### Remineralization

"Remineralization", as used herein, means *in situ* (i.e. in the oral cavity) generation of calcium phosphate on teeth (including layers on teeth from 10 nm to 20 microns, and preferably from 75 nm to 10 microns, and most preferably, from 150 nm to 5 microns thick including all ranges subsumed therein) to reduce the likelihood of tooth sensitivity, tooth decay, regenerate enamel and/or improve the appearance of teeth by whitening through the generation of such new calcium phosphate.

### Deposition Aid

Deposition aid, as used herein, means a material which aids deposition of actives such as calcium silicate and/or particulate tooth whitening agent from the continuous phase of an oral care composition onto the tooth surface during use of the composition.

### Miscellaneous

Except in the examples, or where otherwise explicitly indicated, all numbers in this description indicating amounts of material or conditions of reaction, physical properties of materials and/or use may optionally be understood as modified by the word "about".

All amounts are by weight of the final oral care composition, unless otherwise specified.

It should be noted that in specifying any ranges of values, any particular upper value can be associated with any particular lower value.

For the avoidance of doubt, the word "comprising" is intended to mean "including" but not necessarily "consisting of' or "composed of'. In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

### Summary of the Invention

In a first aspect, the present invention is directed to an oral care composition comprising:
a) calcium silicate;
b) a deposition aid selected from calcium sulfate hemihydrates, calcium dihydrogen phosphate or mixtures thereof; and
c) a physiologically acceptable carrier;
wherein the calcium silicate and the deposition aid is present in a weight ratio from 20:1 to 1:5.

In a second aspect, the present invention is directed to an oral care composition for use in a method for remineralizing and/or reducing sensitivity and/or whitening of teeth of an individual comprising the step of applying the oral care composition of any embodiment of the first aspect to at least one surface of the teeth of the individual.

All other aspects of the present invention will more readily become apparent upon considering the detailed description and examples which follow.

### Detailed Description

Now it has been found expectedly that the tooth remineralization efficacy can be enhanced by using calcium silicate in combination with deposition aids. Besides, the present inventors have further found that such an oral care composition is effective in blocking dentinal tubules to reduce tooth sensitivity which is beneficial for treating tooth hypersensitivity. Additionally, such a composition unexpectedly enhances the deposition of particulate tooth whitening agent on tooth surfaces to provide tooth whitening benefit.

The term "deposition aid" in the context of the present invention generally means a material which aids deposition of actives such as calcium silicate and/or particulate tooth whitening agent from the continuous phase of the composition onto the tooth surface during use of the composition.

### CALCIUM SILICATE

In a preferred embodiment, the calcium silicate used is CaSiO₃ which has low water solubility and is made commercially available under the name Sorbosil CA40 from PQ Corporation. In another preferred embodiment, the calcium silicate is insoluble, present as the composite material calcium oxide-silica (CaO-SiO₂), which is described, for example, in international patent application published as WO 2008/01517(Unilever) which is hereby incorporated by reference in its entirety. For a calcium silicate composite material, the atom ratio of calcium to silicon (Ca:Si) may be from 1:10 to 3:1. The Ca:Si ratio is preferably 1:5 to 2:1, and more preferably, from 1:3 to 2:1, and most preferably, from about 1:2 to 2:1. In another preferred embodiment, the Ca:Si ratio may be from 1:30 to 3:1, preferably from 1:20 to 2:1 and more preferably from 1:10 to 1:1, and most preferably, from 1:7 to 1:1.5.The calcium silicate may comprise mono-calcium silicate, bi-calcium silicate, or tri-calcium silicate. The calcium silicate may be in a crystalline or amorphous state or even in a mesoporous state.

In addition to calcium oxide, silica, the calcium silicate which is not hydrated is substantially free of other elements. The calcium silicate consists of (or at least consists essentially of) calcium oxide, silica.

In an especially preferred embodiment, the calcium silicate is calcium silicate hydrate. The calcium silicate hydrate for use in the present invention comprises at least calcium oxide (CaO), silica (SiO₂) and water. Compared with conventional calcium silicate which are not hydrated, the calcium silicate hydrate comprises the water of hydration in an amount of at least 5% by weight of the calcium silicate hydrate, preferably at least 10%, more preferably at least 15%, even more preferably at least 20% and most preferably at least 25%. The water content is typically no greater than 50% by weight of the calcium silicate hydrate, more preferably no greater than 40%, even more preferably no greater than 35% and most preferably no greater than 30%.

The calcium silicate hydrate preferably comprises at least 20% silica by weight of the calcium silicate hydrate, more preferably at least 30%, more preferably still at least 40% and most preferably at least 55%. The silica content is preferably no greater than 70% by weight of the calcium silicate hydrate, more preferably no greater than 65% and most preferably no greater than 60%.

To provide calcium necessary for remineralization, the calcium silicate hydrate preferably comprises calcium oxide in an amount of at least 5% by weight of the calcium silicate hydrate, more preferably at least 7%, more preferably still at least 10%, even more preferably at least 12% and most preferably at least 15%. The calcium oxide content is typically no greater than 50% by weight of the calcium silicate hydrate, more preferably no greater than 40%, even more preferably no greater than 30% and most preferably no greater than 25%.

The calcium silicate hydrate preferably comprises Ca and Si in an atom ratio (Ca:Si) less than 1:1, more preferably less than 1:1.2, more preferably still from 1:1.5 to 1:4 and most preferably from 1:1.7 to 1:3.

The calcium silicate may be amorphous or at least partly crystalline or mesoporous. The calcium silicate is preferably particulate as this allows for maximum surface area for contact with dental tissue. Thus preferably the composition comprises particles comprising the calcium silicate. More preferably the particles have a weight average particle size of five microns or less, and even more preferably from 10 to 100%, and especially, from 25 to 100%, and most especially, from 70% to 100% by weight of the particles comprising calcium silicate used in this invention have a particle size from 0.1 to 1.5 microns.

In addition to calcium oxide, silica and water, the particles which comprise the calcium silicate hydrate may comprise other components, such as metal cations, anions (such as phosphate) and the like. However, it is preferred that the particles comprise CaO, SiO₂ and water in an amount of at least 70% by weight of the particles, more preferably at least 80%, more preferably still at least 90% and even more preferably at least 95%. Most preferably the particles consist of (or at least consist essentially of) CaO, SiO₂ and water.

Typically, the oral care composition of the present invention comprises from 0.1 to 50% by weight of the calcium silicate, more preferably from 0.2 to 30%, most preferably from 1 to 20%, based on the total weight of the oral care composition and including all ranges subsumed therein.

### DEPOSITION AID

The deposition aid suitable for use in this invention is limited only to the extent that the same may be used in the mouth. In a preferred embodiment, the deposition aid provides plenty of calcium ions in the mouth and is inexpensive and abundant. In an especially preferred embodiment, the deposition aid is calcium salts.

The deposition aid is calcium sulfate hemihydrates, calcium dihydrogen phosphate or mixtures thereof.

It has been discovered that the materials used as deposition aids in this invention is biocompatible, and undergoes rapid reaction with water and complete resorption onto the tooth surface, therefore it can be used to aid the deposition of oral care actives such as calcium silicate and/or particulate tooth whitening agent onto the tooth surface.

Typically, the oral care composition of the present invention comprises from 0.1 to 20% by weight of the deposition aid, more preferably from 0.2 to 15%, more preferably still from 0.5 to 10%, most preferably from 1 to 5%, based on the total weight of the oral care composition and including all ranges subsumed therein.

The oral care composition comprises the calcium silicate and the deposition aid in a weight ratio from 20:1 to 1:5, preferably from 15:1 to 1:3, more preferably from 10:1 to 1:1.

### OPTIONAL COMPONENTS

The oral care composition may further comprise high refractive index particles. The only limitation with respect to the high refractive index particles that may be used in this invention is that the same is suitable for use in the mouth.

In order to provide excellent whitening effect, the particles of the present invention are preferred to have a high refractive index of at least 1.9, more preferably at least 2.0, even more preferably at least 2.2, even more preferably still at least 2.4 and most preferably at least 2.5. The maximum refractive index of the high refractive index particles is not particularly limited but preferably up to 4.0. Typically, the high refractive index particle comprises a material suitable to physically and immediately improve characteristics of teeth and especially whiten teeth. Particularly suitable materials are metal salts and preferred are salts where the metal is selected from zinc (Zn), titanium (Ti), zirconium (Zr) or a combination thereof. Preferably, the metal salts is (or at least comprises) a metal oxide such as titanium dioxide (TiO₂), zinc oxide (ZnO), zirconium dioxide (ZrO₂) or a combination thereof. In addition, the high refractive index particle can also comprise non-metal oxides such as silicon monoxide (SiO).

In a preferred embodiment, the high refractive index particle comprises metal oxides, non-metal oxides or a combination thereof in an amount of at least 50% by weight of the particle and more preferably at least 70%, more preferably still from 80 to 100% and most preferably from 85 to 95%. In an especially preferred embodiment, the high refractive index particle is at least 50% by weight titanium dioxide, and most preferably, from 60 to 100% by weight titanium dioxide, based on total weight of the high refractive index particle and including all ranges subsumed therein. In another especially preferred embodiment, the high refractive index particles are slightly soluble or insoluble in water, but most preferably, insoluble in water.

In a preferred embodiment, the high refractive index particles are composite particles. The refractive index of a composite particle comprising more than one material can be calculated based on the refractive indices and volume fractions of the constituents using effective medium theory, as is described for example in WO 2009/023353.

The composite particle comprises a first component core and a second component coating. Typically, the core of the composite particle comprises a material suitable to physically and immediately improve characteristics of teeth and especially whiten teeth. Particular suitable materials are metal salts and preferred are salts where the metal is selected from zinc (Zn), titanium (Ti), zirconium (Zr) or a combination thereof. Preferably, the metal salt is (or at least comprises) a metal oxide such as titanium dioxide (TiO₂), zinc oxide (ZnO), zirconium dioxide (ZrO₂) or a combination thereof. In addition, the core of the composite particle can also comprise non-metal oxides such as silicon monoxide (SiO).

The core of the composite particle typically makes up from 3 to 98%, and preferably from 6 to 65%, and most preferably from 10 to 55% by weight of the composite particle, based on total weight of the composite particle and including all ranges subsumed therein. In a preferred embodiment, the core comprises metal oxides, non-metal oxides or a combination thereof in an amount of at least 50% by weight of the core and more preferably at least 70%, more preferably still from 80 to 100% and most preferably from 85 to 95%. In an especially preferred embodiment, the core is at least 50% by weight titanium dioxide, and most preferably, from 60 to 100% by weight titanium dioxide, based on total weight of the first component core.

The second component coating comprises material suitable to adhere to tooth enamel, dentin or both. Typically the coating material comprises the element calcium, and optionally, other metals like potassium, sodium, aluminium, magnesium as well as mixtures thereof whereby such optional metals are provided as, for example, sulphates, lactates, oxides, carbonates or silicates. Optionally, the coating material may be aluminium oxide or silica. In a preferred embodiment, the coating material is suitable to provide a biological or chemical improvement to teeth which is long term (e.g., results in hydroxyapatite formation). Preferably, the coating employed comprises at least 50% by weight elemental calcium, and most preferably, at least 65% by weight elemental calcium based on total weight of metal in the coating. In an especially preferred embodiment, the metal in the coating is from 80 to 100% by weight of elemental calcium, based on total weight of metal in the second component coating and including all ranges subsumed therein. In another especially preferred embodiment, the core and the coating are slightly soluble or insoluble in water, but most preferably, insoluble in water.

In an especially desired embodiment, the second component coating can comprise, for example, calcium phosphate, calcium gluconate, calcium oxide, calcium lactate, calcium carbonate, calcium hydroxide, calcium sulphate, calcium carboxymethyl cellulose, calcium alginate, calcium salts of citric acid, calcium silicate, mixture thereof or the like. In another desired embodiment, the calcium source in the coating comprises calcium silicate.

In yet another preferred embodiment, the coating can comprise the element calcium which originates from insoluble calcium silicate, present as the composite material calcium oxide-silica (CaO-SiO₂) as described in international patent applications published as WO 2008/015117 and WO 2008/068248.

When a calcium silicate composite material is employed as coating, the ratio of calcium to silicon (Ca:Si) may be from 1:10 to 3:1. The Ca:Si ratio is preferably from 1:5 to 2:1, and more preferably, from 1:3 to 2:1, and most preferably, from about 1:2 to 2:1. The calcium silicate may comprise mono-calcium silicate, bi-calcium silicate, or tri-calcium silicate whereby ratios of calcium to silicon (Ca:Si) should be understood to be atom ratios.

Usually, at least 30% of the outer surface area of the first component core is coated with the second component coating, preferably at least 50% of the core is coated with the coating, most preferably, from 70 to 100% of the outer surface area of the first component core is coated with the second component coating.

In an especially preferred embodiment, the high refractive index particle is titanium dioxide coated with calcium silicate.

The high refractive index particles according to the present invention can be of different sizes and shapes. The particles may be of spherical, platelet or irregular shape form. The diameter of the high refractive index particles is often from 10 nm to less than 50 microns, and preferably, from 75 nm to less than 10 microns. In an especially preferred embodiment, the diameter of particles is from 100 nm to 5 microns, including all ranges subsumed therein. For composite particles, in a preferred embodiment, at least 40%, and preferably, at least 60%, and most preferably, from 75 to 99.5% of the diameter of the composite particle is the core, including all ranges subsumed therein.

Typically, the oral care composition of the present invention comprises from 0.25 to 60%, and preferably, from 0.5 to 40%, and most preferably, from 1 to 30% by weight of the high refractive index particles, based on the total weight of the oral care composition and including all ranges subsumed therein.

When the high refractive index particles are incorporated into the oral care composition, the relative weight ratio of calcium silicate to high refractive index particles may range from 1:10 to 4:1, preferably from 1:5 to 3:1, most preferably from 1:3 to 2:1.

The oral care composition of the present invention is found to be capable of remineralization of teeth without inclusion of a phosphate source in the composition itself. Without wishing to be bound by theory the present inventors believe that this may be because the calcium silicate in the oral composition of the present invention reacts with phosphate ions in saliva and/or Si-OH groups may have an affinity for Ca ions in teeth. The deposition aid of the present invention enhances the deposition of calcium silicate onto the tooth surface. When high refractive index particles are present in the oral care composition, the remineralization of calcium silicate around the particles further helps the deposition of particles on tooth surface by enhancing their resistance to shear force.

Thus in one embodiment the composition may be substantially free of phosphate source.

For anhydrous compositions (i.e. compositions substantially free of water) or a dual phase hydrous compositions, it is preferred to compound a phosphate source in the composition.
The phosphate source that may be used in this invention is limited only to the extent that the same may be used in a composition suitable for use in the mouth. Illustrative examples of the types of phosphate source suitable for use in this invention include trisodium phosphate, monosodium dihydrogen phosphate, disodium hydrogen phosphate, sodium pyrophosphate, tetrasodium pyrophosphate, sodium hexametaphosphate, tripotassium phosphate, monopotassium dihydrogen phosphate, dipotassium hydrogen phosphate, mixtures thereof or the like. The phosphate source is preferably one which is water soluble.

When used, the phosphate source typically makes up from 0.5 to 22%, and preferably, from 2 to 18%, and most preferably, from 4 to 16% by weight of the oral care composition, based on total weight of the oral care composition and including all ranges subsumed therein. In a preferred embodiment, the phosphate source used is trisodium phosphate and monosodium dihydrogen phosphate at a trisodium phosphate to monosodium dihydrogen phosphate weight ratio of 1:4 to 4:1, preferably 1:3 to 3:1, and most preferably, from 1:2 to 2:1, including all ratios subsumed therein.

The composition of the present invention is an oral care composition and typically comprises physiologically acceptable carrier. The carrier preferably comprises at least surfactant, thickener, humectant or a combination thereof.

Preferably the oral care composition comprises a surfactant. Preferably the composition comprises at least 0.01% surfactant by weight of the composition, more preferably at least 0.1% and most preferably from 0.5 to 7%. Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. More preferably the surfactant comprises or is anionic surfactant. The preferred anionic surfactants are sodium lauryl sulphate and/or sodium dodecylbenzene sulfonate. Most preferably the surfactant is sodium lauryl sulphate.

Thickener may also be used in this invention and is limited only to the extent that the same may be added to a composition suitable for use in the mouth. Illustrative examples of the types of thickeners that may be used in this invention include, sodium carboxymethyl cellulose (SCMC), hydroxyl ethyl cellulose, methyl cellulose, ethyl cellulose, gum tragacanth, gum arabic, gum karaya, sodium alginate, carrageenan, guar, xanthan gum, Irish moss, starch, modified starch, silica based thickeners including silica aerogels, magnesium aluminum silicate (e.g., Veegum), Carbomers (cross-linked acrylates) and mixtures thereof.

Typically, sodium carboxymethyl cellulose and/or a Carbomer is/are preferred. When a Carbomer is employed, those having a weight-average molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

Thickener typically makes up from 0.01 to about 10%, more preferably from 0.1 to 9%, and most preferably, from 1.5 to 8% by weight of the oral care composition, based on total weight of the composition and including all ranges subsumed therein.

When the oral care composition of this invention is a toothpaste or gel, the same typically has a viscosity from about 30,000 to 180,000 mPa.s (centipoise), and preferably, from 60,000 to 170,000 mPa.s (centipoise), and most preferably, from 65,000 to 165,000 mPa.s (centipoise).

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants.

The humectant may be present in the range of from 10 to 90% by weight of the oral care composition. More preferably, the carrier humectant makes up from 25 to 80%, and most preferably, from 45 to 70% by weight of the composition, based on total weight of the composition and including all ranges subsumed therein.

The oral care composition of the present invention may contain a variety of other ingredients which are common in the art to enhance physical properties and performance. These ingredients include antimicrobial agents, anti-inflammatory agents, anti-caries agents, plaque buffers, fluoride sources, vitamins, plant extracts, desensitizing agents, anti-calculus agents, biomolecules, flavors, proteinaceous materials, preservatives, opacifying agents, coloring agents, pH-adjusting agents, sweetening agents, particulate abrasive materials, polymeric compounds, buffers and salts to buffer the pH and ionic strength of the compositions, and mixtures thereof. Such ingredients typically and collectively make-up less than 20% by weight of the composition, and preferably, from 0.0 to 15% by weight, and most preferably, from 0.01 to 12% by weight of the composition, including all ranges subsumed therein.

The oral care composition of this invention can be used in a method of remineralizing and/or reducing sensitivity and/or whitening of teeth of an individual comprising applying the composition to at least one surface of the teeth of the individual. The oral care composition of this invention may additionally or alternatively be for use as a medicament and/or used in the manufacture of a medicament for providing an oral care benefit as described herein, such as for remineralization of the teeth of an individual. Alternatively and preferably, the use is non-therapeutic.

In a preferred embodiment, the oral care composition is a monophase anhydrous composition.

In another preferred embodiment, the oral care composition is a dual-phase composition comprising a calcium source and a phosphate source, wherein the calcium silicate and the deposition aid are present in the calcium phase, which is spatially separated from the phosphate source. The two phases are physically separate from one another by being in independent phases. The delivery of the two independent phases to the teeth may be simultaneous or sequential. In a preferred embodiment, the phases are delivered simultaneously.

Typically, the dual-phase composition is delivered by a dual-tube having a first compartment for the first phase comprising calcium source and a second compartment for the second phase comprising the phosphate source, which allows for co-extrusion of the two phases.

In a preferred embodiment, such a dual-tube has one of the compartments surrounding the other. In such embodiments, one phase is present as a sheath, surrounding the other phase in the core. In an especially preferred embodiment, the phase as the core comprises the calcium source and the sheath phase comprises the phosphate source.

In another preferred embodiment, such a dual-tube has the two compartments side by side within the same tube. In such embodiments, the two phases are extruded from the tube as one, such extrusion being termed "contact extrusion". A pump head may be used in such a dual-tube for squeezing the two phases from the tube as one.

The dual-phase oral care composition may be a gel composition that comprises two independent gel phases, the first comprising the calcium source and the second comprising the phosphate source. The means of delivery may involve a cotton rod, or a tray, onto which the calcium source and the phosphate source are applied, prior to the tray being placed in contact with the teeth.

Typically the composition will be packaged. In tooth paste or gel form, the composition may be packaged in a conventional plastic laminate, metal tube or a single compartment dispenser. The same may be applied to dental surfaces by any physical means, such as a toothbrush, fingertip or by an applicator directly to the sensitive area. In liquid mouthwash form the composition may be packaged in a bottle, sachet or other convenient container.

The composition can be effective even when used in an individual's daily oral hygiene routine. For example, the composition may be brushed onto the teeth and/or be rinsed around the inside of the mouth of the individual. The composition may, for example, be contacted with the teeth for a time period of one second to 20 hours. More preferably from 1 s to 10 hours, more preferably still from 10 s to 1 hour and most preferably from 30 s to 5 minutes. The composition may be used daily, for example for use by an individual once, twice or three times per day. When the oral care composition is a dual-phase composition, the two phases of the composition are mixed during application. The mixed phases are typically left on the teeth for from 3 minutes to 10 hours, more preferably from 3 minutes to 8 hours. The application may be carried out daily.

The following examples are provided to facilitate an understanding of the present invention.

### Examples

### Example 1

This example demonstrates the improvement of deposition effect on tooth surface by using calcium silicate in combination with deposition aids. All ingredients are expressed by weight percent of the total formulation, and as level of active ingredient.

**TABLE 1**

| Ingredient | Samples | | |
|---|---|---|---|
| | **1** | **2** | **3** |
| Calcium silicate^{a} | 15 | 10 | 10 |
| Calcium sulfate hemihydrate | - | 5 | - |
| Calcium dihydrogen phosphate | - | - | 5 |
| Glycerine | 85 | 85 | 85 |

| | | | |
|---|---|---|---|
| a. Commercially available calcium silicate (CaSiO₃) from P.Q.Corporation (Sorbosil CA40) | | | |

### Methods

To evaluate deposition on tooth surface, the test sample was mixed with water at a ratio of 5 g to 10 mL water to make a slurry. Sample 1 comprising commercial calcium silicate (CS) and glycerine was used as control.

The bovine enamel blocks were treated with different slurries via brushing following the same protocol. The enamel blocks were brushed with the slurry under a tooth brushing machine equipped with toothbrushes. The load of the tooth brushing was 170 g +/-5 g and the automatic brushing operated at a speed of 150 rpm. After brushing for 1 min, the enamel blocks were rinsed with distilled water. Then the enamel blocks were soaked in simulated oral fluid (SOF) for at least 3 hours under the condition of a shaking water bath at 37°C and 60.0 rpm. The enamel blocks were characterized after 6 and 14 times brushing.

Simulated oral fluid was made by combining the ingredients in Table 2:

**TABLE 2**

| Ingredient | Amount/g |
|---|---|
| NaCl | 16.07 |
| NaHCO₃ | 0.7 |
| KCl | 0.448 |
| K₂HPO₄*3H₂O | 3.27 |
| MgCl₂*6H₂O | 0.622 |
| 1M HCl | 40ml |
| CaCl₂ | 0.1998 |
| Na₂SO₄ | 0.1434 |
| Buffer | Adjust pH to 7.0 |
| Water | Balance to 2L |

### Results

After 6 and 14 times of brushing, Scanning Electron Microscopy (SEM) images of the enamel block surfaces were taken. From the SEM images, it was apparent that Samples 2 and 3 gave better and denser deposition on tooth surface than Sample 1 which comprises only the commercial CS. The corresponding cross-section SEM images after 6 times brushing showed that a new layer was formed on the surface of the enamel blocks after treatment with Sample 2, while Sample 1 treated enamel blocks did not have a layer formed on their surfaces. Analysis using EDX (Energy Dispersive X-ray Spectroscopy) indentified the elements of Si, Ca and P within the new layer, indicating that CS deposited on tooth surface and induced remineralization.

### Example 2

This example demonstrates the improved blockage of dentinal tubules by using calcium silicate in combination with a deposition aid. All ingredients are expressed by weight percent of the total formulation, and as level of active ingredient.

**TABLE 3**

| Ingredient | **Samples** | | | | |
|---|---|---|---|---|---|
| | **4** | **5** | **6** | **7** | **8** |
| Glycerin | Balance | Balance | Balance | Balance | Balance |
| Sodium monofluorophosphate | 1.11 | 1.11 | 1.11 | 1.11 | 1.11 |
| Sodium saccharin | 0.25 | 0.25 | 0.25 | 0.25 | 0.25 |
| Calcium silicate^{a} | 15.00 | 15.00 | 15.00 | - | - |
| Sodium lauryl sulfate (70%) | 2.00 | 2.00 | 2.00 | 2.00 | 2.00 |
| Monosodium dihydrogen phosphate | 3.20 | 3.20 | 3.20 | 3.20 | 3.20 |
| Trisodium phosphate | 3.80 | 3.80 | 3.80 | 3.80 | 3.80 |
| Silica abrasive^{b} | 6.00 | 6.00 | 6.00 | 6.00 | 6.00 |
| Silica abrasive^{c} | 0.50 | 0.50 | 0.50 | 0.50 | 0.50 |
| Thickener | 0.10 | 0.10 | 0.10 | 0.10 | 0.10 |
| Cacium sulfate hemihydrate | - | 5.00 | - | 5.00 | - |
| Calcium dihydrogen phosphate | - | - | 2.00 | - | 2.00 |
| Flavor | 1.10 | 1.10 | 1.10 | 1.10 | 1.10 |

| | | | | | |
|---|---|---|---|---|---|
| b. Commercially available silica abrasive (SiO₂) from J.M. Corporation (Zeodent 124) c. Commercially available silica abrasive (SiO₂) from PQ Corporation (Sorbosil AC43) | | | | | |

### Methods

To evaluate blockage efficacy of dentinal tubules, the test sample was mixed with water at a ratio of 1g to 2mL water to make a slurry. Sample 4 comprising only commercial calcium silicate was used as control.

Dentine samples were treated with different slurries via brushing following the same protocol. The dentine samples were brushed with the slurry under a tooth brushing machine equipped with toothbrushes. The load of the tooth brushing was 170 g +/-5 g and the automatic brushing operated at a speed of 150 rpm. After brushing for 1 min, the dentine samples were soaked in SOF for 1 min. Then the dentine samples were rinsed with distilled water and placed in SOF under the condition of a shaking water bath at 37°C and 60.0 rpm. After soaking for about 3 to 4 hours, the dentine samples were brushed with the slurry by machine using the same procedure as in the first step. The brushing was repeated three times for one day, then the dentine samples were kept in SOF ovemight(>12 hours) in a shaking water bath at 37°C to mimic oral environment. The dentine samples were brushed for 7 times, 14 times and 28 times. After 28 times brushing (mimic 2 weeks of twice-daily tooth brushing), the dentine samples were soaked in 0.1% citric acid for 5 mins to test the acid resistance of the deposition layer.

### Results

After 7 brushings, SEM (Scanning Electron Microscopy) images of the dentine samples were taken. It appeared that the dentine samples treated with Samples 5 and 6 showed that the tubules are filled significantly better compared to Sample 4 comprising only calcium silicate. For Sample 4, it can be seen that lots of dentinal tubules were still open. Samples 5 and 6 also showed significantly better tubule blockage efficacy than Samples 7 and 8, which comprises the deposition aid only. For Samples 7 and 8, it can be seen that lots of dentinal tubules were still open. While for Samples 5 and 6, the tubules were extensively filled and sealed.

The corresponding cross-section SEM images after 28 times brushing showed that a new layer was formed on the surface of the dentine samples for Samples 5 and 6. Additionally, it can be seen that the materials were penetrated into the dentinal tubules and formed tubule plugs.

### Example 3

This example demonstrates the improvement of tooth whitening efficacy by using high refractive index particles in the presence of calcium silicate (CS) and a deposition aid.

**TABLE 4**

| Ingredient | **Samples** | | | | | | | |
|---|---|---|---|---|---|---|---|---|
| | **9** | **10** | **11** | **12** | **13** | **14** | **15** | **16** |
| Calcium silicate^{a} | 50 | 33.3 | 33.3 | 33.3 | 33.3 | 33.3 | - | - |
| Calcium silicate coated titanium dioxide^{d} | 50 | 50 | 50 | 50 | 50 | 50 | 50 | 50 |
| Calcium carbonate | - | 16.7 | - | - | - | - | - | - |
| Calcium sulfate | - | - | 16.7 | - | - | - | - | - |
| Calcium sulfate hemihydrates | - | - | - | 16.7 | - | - | 50 | - |
| Calcium dihydrogen phosphate | - | - | - | - | 16.7 | - | - | 50 |
| Calcium monohydrogen phosphate | - | - | - | - | - | 16.7 | - | - |

| | | | | | | | | |
|---|---|---|---|---|---|---|---|---|
| d. Commercially available calcium silicate coated titanium dioxide from KOBO Products Inc | | | | | | | | |

### Methods

### a. Brushing protocol for samples comprising CS and a deposition aid (except for calcium dihydrogen phosphate)

To investigate the whitening effect of the samples, fresh slurries were prepared by mixing test sample with water at a ratio of 1.5 g to 10 mL water for 20 seconds, and used immediately.

The bovine enamel blocks were treated with different slurries via brushing following the same protocol. The enamel blocks were brushed with the slurry under a tooth brushing machine equipped with toothbrushes. The load of the tooth brushing was 170 g +/-5 g and the automatic brushing operated at a speed of 150 rpm. After brushing for 1 min, the enamel blocks were rinsed with distilled water twice and soaked in SOF for 3 hours under the condition of a shaking water bath at 37°C and 60.0 rpm. The same brushing step was repeated for 3 times. Then the tooth blocks were kept in SOF overnight (>12 hours) in a shaking water bath at 37°C to mimic oral environment. These steps are considered as a whole treatment cycle within one day. After one and three cycles, the changes in whiteness was recorded using a Konica Minolta-2600D colorimeter. The WIO index is an index which has been optimized specifically for the evaluation of whiteness in teeth (as described in Journal of Dentistry, volume 36, Supplement 1, 2008, pages 2 to 7). The changes in WIO values (ΔWIO) were measured by colorimeter and statistically analyzed.

### b. Brushing protocol for sample comprising CS and calcium dihydrogen phosphate

Due to the quick reaction of calcium dihydrogen phosphate and water, the brushing protocol for sample comprising CS and calcium dihydrogen phosphate has been modified.

Fresh slurry was prepared by mixing 0.5 g CS and 0.75 g calcium silicate coated titanium dioxide with 10 mL water. 0.25 g calcium dihydrogen phosphate was added directly onto the surface of the enamel blocks. The slurry of CS and calcium silicate coated titanium dioxide was poured onto the surface of the enamel blocks. *In-situ* brushing was initiated and lasted for 1 min. Then the enamel blocks were rinsed with distilled water twice and soaked in SOF for 3 hours under the condition of a shaking water bath at 37°C and 60.0 rpm. The same brushing step was repeated for 3 times. Then the enamel blocks were kept in SOF overnight (>12 hours) in a shaking water bath at 37°C to mimic oral environment. These steps are considered as a whole treatment cycle within one day. After one and three cycles, the changes in WIO values (ΔWIO) were measured by colorimeter and statistically analyzed.

The results after one cycle and three cycles treatment are summarized in Table 5 (error represents 95% confidence interval for duplicate measurements).

**TABLE 5**

| Change in WIO | **Samples** | | | | | | | |
|---|---|---|---|---|---|---|---|---|
| | **9** | **10** | **11** | **12** | **13** | **14** | **15** | **16** |
| One cycle | 2.63±0.811 | 3.09±1.082 | 2.88±2.244 | 12.02±3.996 | 10.83±2.180 | 3.51±1.611 | 0.91±0.956 | 1.37±1.013 |
| Three cycles | 4.93±2.895 | 4.86±3.852 | 3.93±2.446 | 11.94±5.407 | 20.72±5.914 | 2.87±2.943 | -1.24±0.839 | 0.64±1.806 |

### Results

Samples 12 and 13, which comprise calcium sulfate hemihydrates and calcium dihydrogen phosphate respectively, showed significantly better tooth whitening effect than other samples.

After three cycles treatment, SEM (Scanning Electron Microscopy) images of the enamel blocks were taken. It appeared that the enamel blocks treated with Samples 12 and 13 showed the formation of a new layer on tooth surfaces. Analysis using EDX indentified the elements of Ti, Si, Ca and P within the new layer, indicating the deposition of composite particles on tooth surface.

### Example 4

This example demonstrates the effect of the concentration of deposition aids in the composition on the tooth whitening efficacy.

**TABLE 6**

| Ingredient | Samples | | | | |
|---|---|---|---|---|---|
| | **17** | **18** | **19** | **20** | **21** |
| Glycerin | 63.69 | 62.69 | 61.69 | 60.69 | 58.69 |
| Sodium monofluorophosphate | 1.11 | 1.11 | 1.11 | 1.11 | 1.11 |
| Sodium saccharin | 0.25 | 0.25 | 0.25 | 0.25 | 0.25 |
| Calcium silicate^{a} | 10.00 | 10.00 | 10.00 | 10.00 | 10.00 |
| Calcium silicate coated titanium dioxide^{d} | 15.00 | 15.00 | 15.00 | 15.00 | 15.00 |
| Silica abrasive | 6.00 | 6.00 | 6.00 | 6.00 | 6.00 |
| Thickener | 0.75 | 0.75 | 0.75 | 0.75 | 0.75 |
| Calcium dihydrogen phosphate | - | 1.00 | 2.00 | 3.00 | 5.00 |
| Sodium lauryl sulfate (70%) | 2.00 | 2.00 | 2.00 | 2.00 | 2.00 |
| Flavor | 1.20 | 1.20 | 1.20 | 1.20 | 1.20 |

### Methods

To investigate the whitening effect of the samples, toothpaste samples and water were *in situ* mixed into aqueous slurries. The bovine enamel blocks were treated with different slurries via brushing following the same protocol. The enamel blocks were brushed with the slurry under a tooth brushing machine equipped with toothbrushes. The load of the tooth brushing was 170 g +/-5 g and the automatic brushing operated at a speed of 150 rpm. 4 g of toothpaste sample was added directly onto the enamel surface. Once 8 mL water was added, automatic tooth brushing by machine was initiated. After brushing for 1 min, the enamel blocks were left for 1 min soaking in the slurry. Then the enamel blocks were rinsed with distilled water and soaked in SOF for 3 hours under the condition of a shaking water bath at 37°C and 60.0 rpm. The same brushing step was repeated for 2 times each day. Then the enamel blocks were kept in SOF overnight (>12 hours) in a shaking water bath at 37°C to mimic oral environment. In total, 28 times brushing was performed to simulate 2 weeks of twice daily brushing. After 14 and 28 times toothpaste brushing, the changes in WIO values (ΔWIO) were measured by colorimeter and statistically analyzed.

The results after 14 and 28 times treatment are summarized in Table 7 (error represents 95% confidence interval for duplicate measurements).

**TABLE 7**

| Change in WIO | Samples | | | | |
|---|---|---|---|---|---|
| | **17** | **18** | **19** | **20** | **21** |
| 14 times | 4.54±4.32 | 5.82±2.79 | 8.54±4.87 | 7.18±4.63 | 7.52±3.44 |
| 28 times | 5.40±2.82 | 5.89±3.75 | 10.27±5.10 | 7.63±3.69 | 9.51±3.56 |

### Results

It showed that samples 18 to 21 comprising calcium dihydrogen phosphate exhibited better tooth whitening effect than sample 17. Besides, sample 19 comprising 2% by weight of calcium dihydrogen phosphate showed superior tooth whitening efficacy to other samples.

### Example 5

This example demonstrates the improvement of tooth whitening efficacy by incorporation of phosphate source.

**TABLE 8**

| Ingredient | Samples | |
|---|---|---|
| | **22** | **23** |
| Glycerin | 60.44 | 61.94 |
| Sodium monofluorophosphate | 1.11 | 1.11 |
| Sodium saccharin | 0.25 | 0.25 |
| Calcium silicate^{a} | 10.00 | 10.00 |
| Calcium silicate coated titanium dioxide^{b} | 15.00 | 15.00 |
| Silica abrasive | 6.00 | 6.00 |
| Thickener | 0.50 | 0.50 |
| Calcium dihydrogen phosphate | 2.00 | 2.00 |
| Sodium lauryl sulfate (70%) | 2.00 | 2.00 |
| Tri sodium phosphate | 0.81 | - |
| Sodium dihydrogen phosphate | 0.69 | - |
| Flavor | 1.20 | 1.20 |

### Methods

To investigate the whitening effect of the samples, toothpaste samples and water were *in situ* mixed into aqueous slurries.

The bovine enamel blocks were treated with different slurries via brushing following the same protocol. The enamel blocks were brushed with the slurry under a tooth brushing machine equipped with toothbrushes. The load of the tooth brushing was 170 g +/-5 g and the automatic brushing operated at a speed of 150 rpm. 4 g of toothpaste sample was added directly onto the enamel surface. Once 8 mL water was added, automatic tooth brushing by machine was initiated. After brushing for 1 min, the enamel blocks were left for 1 min soaking in the slurry. Then the enamel blocks were rinsed with distilled water and soaked in SOF for 3 hours under the condition of a shaking water bath at 37°C and 60.0 rpm. The same brushing step was repeated for 2 times each day. Then the enamel blocks were kept in SOF overnight (>12 hours) in a shaking water bath at 37°C to mimic oral environment. In total, 28 times brushing was performed to simulate 2 weeks of twice daily brushing. After 1, 14 and 28 times toothpaste brushing, the changes in WIO values (ΔWIO) were measured by colorimeter and statistically analyzed.

The results after 1, 14 and 28 times treatment are summarized in Table 9 (error represents 95% confidence interval for duplicate measurements).

**TABLE 9**

| Change in WIO | Samples | |
|---|---|---|
| | **22** | **23** |
| 1 time | 6.70±1.39 | 5.58±2.07 |
| 14 times | 7.25±2.34 | 5.75±4.35 |
| 28 times | 9.02±3.13 | 6.26±2.83 |

### Results

After 14 times brushings, Sample 22 comprising both calcium dihydrogen phosphate and phosphate source showed better tooth whitening efficacy compared to Sample 23 which comprises only calcium dihydrogen phosphate. After 28 times brushings, Sample 22 showed significantly better tooth whitening efficacy than Sample 23.

### Example 6

This example demonstrates the improvement of tooth whitening efficacy by using a combination of CS, a deposition aid and high refractive index particles in a dual-phase composition.

**TABLE 10**

| Ingredient | Percent by Weight | | | |
|---|---|---|---|---|
| | Sample 24 | | Sample 25 | |
| | Calcium Phase | Phosphate Phase | Calcium Phase | Phosphate Phase |
| Calcium silicate^{a} | 10 | - | 10 | - |
| Sodium dihydrogen phosphate | - | 0.69 | - | 0.69 |
| Tri sodium phosphate | - | 0.81 | - | 0.81 |
| Thickener | 0.3 | - | 0.3 | - |
| Glycerol | 69.7 | - | 67.7 | - |
| Calcium dihydrogen phosphate | - | - | 2 | - |
| Calcium silicate coated titanium dioxide^{d} | 20 | - | 20 | - |
| Cellulose gum | - | 1 | - | 1 |
| Ethylhexyl glycerine | - | 0.3 | - | 0.3 |
| Benzyl alcohol | - | 0.3 | - | 0.3 |
| Sodium saccharin | - | 0.3 | - | 0.3 |
| Water | - | 96.6 | - | 96.6 |

### Methods

To investigate the tooth whitening effect of the samples, the calcium and phosphate phases of the samples were first mixed in a weight ratio of 1:1 to form a mixture.

Human extracted teeth were cleaned using prophylaxis paste and placed in the mixture. Then the teeth were gently agitated for 30 s and incubated in the mixture for 15 mins under the condition of a shaking water bath at 37°C and 60.0 rpm. After 15 mins, the teeth were placed in water and agitated on flat bed shaker for 1 min at 150.0 rpm. Then the teeth were incubated in SOF for 5.5 hours under the condition of a shaking water bath at 37°C and 60.0 rpm. After that, the teeth were water brushed for 1 min. These steps are considered as a whole treatment. The human teeth were treated for one, three and five times. After one, three and five times treatment, the changes in WIO values (ΔWIO) were measured by colorimeter and statistically analyzed.

The results after one, three and five times treatment are summarized in Table 11 (error represents standard error for duplicate measurements).

**TABLE 11**

| Change in WIO | Samples | |
|---|---|---|
| | **24** | **25** |
| One time | 6.94±1.52 | 15.71±2.75 |
| Three times | 9.79±1.46 | 33.05±3.24 |
| Five times | 14.24±3.39 | 41.51±3.46 |

### Results

Sample 25 comprising calcium dihydrogen phosphate showed significantly better tooth whitening efficacy compared to Sample 24.

### Example 7

This example shows oral care compositions consistent with this invention.

**TABLE 12**

| Ingredient | Percent by weight |
|---|---|
| Glycerin | 47.44 |
| PEG3000 | 1.50 |
| PEG400 | 10.50 |
| Sodium monofluorophosphate | 1.11 |
| Sodium saccharin | 0.25 |
| Calcium silicate^{a} | 10.00 |
| Calcium silicate coated titanium dioxide^{d} | 15.00 |
| Silica abrasive | 6.00 |
| Calcium dihydrogen phosphate | 5.00 |
| Sodium lauryl sulfate (70%) | 2.00 |
| Flavor | 1.20 |

**TABLE 13**

| Ingredient | Percent by weight | |
|---|---|---|
| | Calcium Phase | Phosphate Phase |
| Calcium silicate^{a} | 10 | - |
| Sodium dihydrogen phosphate | - | 0.69 |
| Tri sodium phosphate | - | 0.81 |
| Thickener | 0.3 | - |
| Glycerol | 67.7 | - |
| Calcium dihydrogen phosphate | 2 | - |
| Calcium silicate coated titanium dioxide^{d} | 20 | - |
| Cellulose gum | - | 1 |
| Ethylhexyl glycerine | - | 0.3 |
| Benzyl alcohol | - | 0.3 |
| Sodium saccharin | - | 0.3 |
| Water | - | 96.6 |

## Claims

1. An oral care composition comprising:
a) calcium silicate;
b) a deposition aid selected from calcium sulfate hemihydrates, calcium dihydrogen phosphate or mixtures thereof; and
c) a physiologically acceptable carrier;
wherein the calcium silicate and the deposition aid is present in a weight ratio from 20:1 to 1:5.

2. The oral care composition according to claim 1, wherein the calcium silicate is calcium silicate hydrate which comprises water of hydration in an amount of from 5 to 50% by weight of the calcium silicate hydrate.

3. The oral care composition according to claim 1 or claim 2, wherein the calcium silicate is present in an amount from 0.1 to 50%, preferably 1 to 20% by weight of the composition.

4. The oral care composition according to any of the preceding claims, wherein the calcium silicate and the deposition aid is present in a weight ratio from 10:1 to 1: 1.

5. The oral care composition according to any of the preceding claims, wherein the composition further comprises high refractive index particles having a refractive index ranging from 1.9 to 4.0, preferably ranging from 2.5 to 4.0.

6. The oral care composition according to claim 5, wherein the high refractive index particles are zinc oxide, titanium dioxide, zirconium dioxide or a mixture thereof, preferably titanium dioxide.

7. The oral care composition according to claim 5 or 6, wherein the high refractive index particles are composite particles comprising a first component core comprising a metal compound, preferably the metal is selected from zinc, titanium, zirconium or a mixture thereof, and a second component coating comprising the element calcium, and optionally, potassium, sodium, magnesium, aluminium or a mixture thereof.

8. The oral care composition according to claim 7, wherein the high refractive index particle is titanium dioxide coated with calcium silicate.

9. The oral care composition according to any of the claims 5 to 8, wherein the high refractive index particle is present in an amount from 1 to 30% by weight of the composition.

10. The oral care composition according to any of the preceding claims, wherein the composition further comprises a phosphate source selected from trisodium phosphate, monosodium dihydrogen phosphate, disodium hydrogen phosphate, sodium pyrophosphate, tetrasodium pyrophosphate, sodium hexametaphosphate, tripotassium phosphate, monopotassium dihydrogen phosphate, dipotassium hydrogen phosphate or a mixture thereof, preferably trisodium phosphate, monosodium dihydrogen phosphate or mixtures thereof.

11. The oral care composition according to any of the preceding claims, wherein the water content of the composition is less than 1.5%, preferably from 0.0 to 0.75% by total weight of the oral care composition.

12. The oral care composition according to any of the preceding claims, wherein the oral care composition is a monophase anhydrous composition.

13. The oral care composition according to claim 10, wherein the composition is a dual-phase composition comprising a calcium phase and a phosphate phase, wherein the calcium silicate and the deposition aid are present in the calcium phase.

14. The oral care composition according to claim 13, wherein the calcium phase and the phosphate phase are physically separate prior to the use of the composition.

15. An oral care composition according to any one of claims 1 to 14 for use in a method for remineralizing and/or reducing sensitivity and/or whitening the teeth of an individual comprising the step of applying the composition to at least one surface of the teeth of the individual.

## Patentansprüche

1. Mundpflegezusammensetzung, umfassend:
a) Calciumsilicat;
b) ein Abscheidungshilfsmittel, ausgewählt unter Calciumsulfathemihydraten, Calciumdihydrogenphosphat oder Mischungen davon; und
c) einen physiologisch verträglichen Träger;
wobei das Calciumsilicat und das Abscheidungshilfsmittel in einem Gewichtsverhältnis von 20:1 bis 1:5 vorliegen.

2. Mundpflegezusammensetzung nach Anspruch 1, wobei das Calciumsilicat Calciumsilicathydrat ist, das Hydratwasser in einer Menge von 5 bis 50 Gewichts-% des Calciumsilicathydrats umfasst.

3. Mundpflegezusammensetzung nach Anspruch 1 oder Anspruch 2, wobei das Calciumsilicat in einer Menge von 0,1 bis 50 Gewichts-%, vorzugsweise von 1 bis 20 Gewichts-% der Zusammensetzung vorliegt.

4. Mundpflegezusammensetzung nach irgendeinem der vorhergehenden Ansprüche, wobei das Calciumsilicat und das Abscheidungshilfsmittel in einem Gewichtsverhältnis von 10:1 bis 1:1 vorliegen.

5. Mundpflegezusammensetzung nach irgendeinem der vorhergehenden Ansprüche, wobei die Zusammensetzung ferner Teilchen mit hohem Brechungsindex umfasst, die einen Brechungsindex aufweisen, der von 1,9 bis 4,0, vorzugsweise von 2,5 bis 4,0, reicht.

6. Mundpflegezusammensetzung nach Anspruch 5, wobei die Teilchen mit hohem Brechungsindex Zinkoxid, Titandioxid, Zirconiumdioxid oder eine Mischung davon, vorzugsweise Titandioxid, darstellen.

7. Mundpflegezusammensetzung nach Anspruch 5 oder 6, wobei die Teilchen mit hohem Brechungsindex Verbundteilchen sind, die einen Kern als ersten Bestandteil, umfassend eine Metallverbindung, wobei das Metall vorzugsweise unter Zink, Titan, Zirconium oder einer Mischung davon ausgewählt ist, und eine Beschichtung als zweiten Bestandteil, umfassend das Element Calcium und gegebenenfalls Kalium, Natrium, Magnesium, Aluminium oder eine Mischung davon, umfassen.

8. Mundpflegezusammensetzung nach Anspruch 7, wobei das Teilchen mit hohem Brechungsindex Titandioxid, beschichtet mit Calciumsilicat, darstellt.

9. Mundpflegezusammensetzung nach irgendeinem der Ansprüche 5 bis 8, wobei das Teilchen mit hohem Brechungsindex in einer Menge von 1 bis 30 Gewichts-% der Zusammensetzung vorliegt.

10. Mundpflegezusammensetzung nach irgendeinem der vorhergehenden Ansprüche, wobei die Zusammensetzung ferner eine Phosphatquelle, ausgewählt unter Trinatriumphosphat, Mononatriumdihydrogenphosphat, Dinatriumhydrogenphosphat, Natriumpyrophosphat, Tetranatriumpyrophosphat, Natriumhexametaphosphat, Trikaliumphosphat, Monokaliumdihydrogenphosphat, Dikaliumhydrogenphosphat oder einer Mischung davon, vorzugsweise Trinatriumphosphat, Mononatriumdihydrogenphosphat oder Mischungen davon, umfasst.

11. Mundpflegezusammensetzung nach irgendeinem der vorhergehenden Ansprüche, wobei der Wassergehalt der Zusammensetzung weniger als 1,5%, vorzugsweise von 0,0 bis 0,75%, bezogen auf das Gesamtgewicht der Mundpflegezusammensetzung, beträgt.

12. Mundpflegezusammensetzung nach irgendeinem der vorhergehenden Ansprüche, wobei die Mundpflegezusammensetzung eine einphasige wasserfreie Zusammensetzung ist.

13. Mundpflegezusammensetzung nach Anspruch 10, wobei die Zusammensetzung eine zweiphasige Zusammensetzung ist, die eine Calciumphase und eine Phosphatphase umfasst, wobei das Calciumsilicat und das Abscheidungshilfsmittel in der Calciumphase vorliegen.

14. Mundpflegezusammensetzung nach Anspruch 13, wobei die Calciumphase und die Phosphatphase vor der Verwendung der Zusammensetzung physikalisch getrennt sind.

15. Mundpflegezusammensetzung nach irgendeinem der Ansprüche 1 bis 14 zur Verwendung in einem Verfahren zum Remineralisieren und/oder Reduzieren der Empfindlichkeit und/oder zum Aufhellen von Zähnen eines Individuums, umfassend den Schritt des Aufbringens der Zusammensetzung auf mindestens eine Oberfläche der Zähne des Individuums.

## Revendications

1. Composition pour le soin oral comprenant :
a) du silicate de calcium ;
b) un aide au dépôt choisi parmi des hémihydrates de sulfate de calcium, du dihydrogénophosphate de calcium ou des mélanges de ceux-ci ; et
c) un support physiologiquement acceptable ;
dans laquelle le silicate de calcium et l'aide au dépôt sont présents dans un rapport massique de 20:1 à 1:5.

2. Composition pour le soin oral selon la revendication 1, dans laquelle le silicate de calcium est un hydrate de silicate de calcium qui comprend de l'eau d'hydratation dans une quantité de 5 à 50 % en masse de l'hydrate de silicate de calcium.

3. Composition pour le soin oral selon la revendication 1 ou la revendication 2, dans laquelle le silicate de calcium est présent dans une quantité de 0,1 à 50 %, de préférence de 1 à 20 % en masse de la composition.

4. Compostion pour le soin oral selon l'une quelconque des revendications précédentes, dans laquelle le silicate de calcium et l'aide au dépôt sont présents dans un rapport massique de 10:1 à 1:1.

5. Composition pour le soin oral selon l'une quelconque des revendications précédentes, dans laquelle la composition comprend de plus des particules d'indice de réfraction élevé présentant un indice de réfraction de 1,9 à 4,0, de préférence de 2,5 à 4,0.

6. Composition pour le soin oral selon la revendication 5, dans laquelle les particules d'indice de réfraction élevé sont l'oxyde de zinc, dioxyde de titane, dioxyde de zirconium ou un mélange de ceux-ci, de préférence le dioxyde de titane.

7. Composition pour le soin oral selon la revendication 5 ou 6, dans laquelle les particules d'indice de réfraction élevé sont des particules composites comprenant un noyau de premier constituant comprenant un composé de métal, le métal est de préférence choisi parmi le zinc, titane, zirconium ou un mélange de ceux-ci, et un revêtement de second constituant comprenant l'élément calcium, et éventuellement, du potassium, sodium, magnésium, aluminium ou un mélange de ceux-ci.

8. Composition pour le soin oral selon la revendication 7, dans laquelle la particule d'indice de réfraction élevé est du dioxyde de titane revêtu de silicate de calcium.

9. Composition pour le soin oral selon l'une quelconque des revendications 5 à 8, dans laquelle la particule d'indice de réfraction élevé est présente dans une quantité de 1 à 30 % en masse de la composition.

10. Composition pour le soin oral selon l'une quelconque des revendications précédentes, dans laquelle la composition comprend de plus une source de phosphate choisie parmi le phosphate de trisodium, dihydrogénophosphate de monosodium, hydrogénophosphate de disodium, pyrophosphate de sodium, pyrophosphate de tétrasodium, hexamétaphosphate de sodium, phosphate de tripotassium, dihydrogénophosphate de monopotassium, hydrogénophosphate de dipotassium ou un mélange de ceux-ci, de préférence phosphate de trisodium, dihydrogénophosphate de monosodium ou mélanges de ceux-ci.

11. Composition pour le soin oral selon l'une quelconque des revendications précédentes, dans laquelle la teneur en eau de la composition est inférieure à 1,5 %, de préférence de 0,0 à 0,75 % en masse totale de la composition pour le soin oral.

12. Composition pour le soin oral selon l'une quelconque des revendications précédentes, dans laquelle la composition pour le soin oral est une composition anhydre de monophase.

13. Composition pour le soin oral selon la revendication 10, dans laquelle la composition est une composition de phase double comprenant une phase de calcium et une phase de phosphate, dans laquelle le silicate de calcium et l'aide au dépôt sont présents dans la phase de calcium.

14. Composition pour le soin oral selon la revendication 13, dans laquelle la phase de calcium et la phase de phosphate se séparent physiquement avant l'utilisation de la composition.

15. Composition pour le soin oral selon l'une quelconque des revendications 1 à 14 pour une utilisation dans un procédé de reminéralisation et/ou réduction de la sensibilité et/ou blanchiment des dents d'un individu comprenant l'étape d'application de la composition sur au moins une surface des dents de l'individu.
